# EUROPEAN PATENT APPLICATION

(11) **EP 1 808 426 A1**
(43) Date of publication of application: **18.07.2007**
(21) Application number: 06000825.7
(22) Date of filing: 16.01.2006
(51) Int. Cl.: C07C 29/42, C07C 33/044, C11D 3/20, B01F 17/38

(54) **Process for the preparation of alkynediols**

(71) Applicant: DSM IP Assets B.V., 6411 TE Heerlen (NL)
(72) Inventor: Bonrath, Walter., DE 79115 Freiburg (DE); Gloor, Arnold., CH 4104 Oberwil (CH); Pace, Francesco., CH 4310 Rheinfelden (CH)
(74) Representative: Steck, Melanie

(57) **Abstract**

The present invention relates to a novel process for the manufacture of alkinediols of the formula I 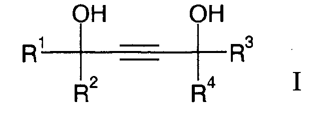 wherein ketones of the formula II 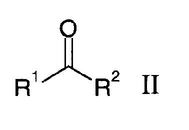 are reacted with alkinols of the formula III 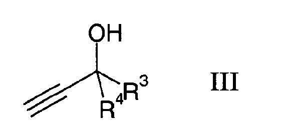 in liquid ammonia in the presence of potassium hydroxide, wherein R¹ and R³ are independently from each other a linear, branched or cyclic C₂₋₂₅-carbon-hydrogen moiety, optionally containing 1 to 10 non-conjugated double-bond(s), and R² and R⁴ are independently from each other linear C₁₋₄-alkyl.

The present invention also refers to the novel use of the alkindiols as emulgator, surfactant, dispersant, anti-foaming non-ionic agent, viscosity stabilizer, antistatic lubricant in the spinning of fibres, solvent, flavour, fragrance, wetting agent, sealing material or intermediate thereof and to the following novel alkindiols: 4,7-dihydroxy-2,4,7,9-tetramethyl-5-decine, 10, 13-dihydroxy-2,6,10,13,17,21-hexamethyl-11-docosine and 14, 17-dihydroxy-2,6,10,14,17,21,25,29-octamethyl-15-triacontine.

## Description

The present invention relates to a novel process for the manufacture of alkinediols of the formula I 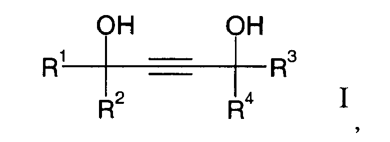 as well as to their novel use as emulgator, surfactant, dispersant, anti-foaming non-ionic agent, viscosity stabilizer, antistatic lubricant in the spinning of fibres, solvent, flavour, fragrance, wetting agent, sealing material or intermediate thereof and to the following novel alkindiols: 4,7-dihydroxy-2,4,7,9-tetramethyl-5-decine, 10, 13-dihydroxy-2,6,10,13,17,21-hexamethyl-11-docosine and 14, 17-dihydroxy-2,6,10,14,17,21,25,29-octamethyl-15-triacontine.

According to the process of the present invention alkinediols of the formula I are obtained 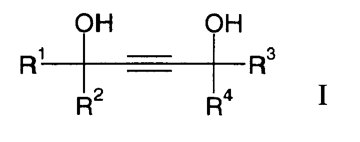 by reacting ketones of the formula II 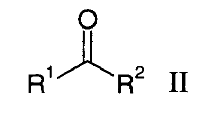 with alkinols of the formula III 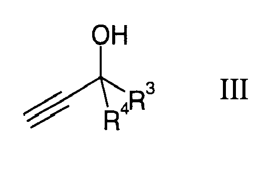 in liquid ammonia in the presence of potassium hydroxide, wherein R¹ and R³ are independently from each other a linear, branched or cyclic C₂₋₂₅-carbon-hydrogen moiety, optionally containing 1 to 10 non-conjugated double-bond(s), and R² and R⁴ are independently from each other linear C₁₋₄-alkyl.

The term "linear, branched or cyclic C₂₋₂₅-carbon-hydrogen moiety" encompasses linear alkyl, branched alkyl, cycloalkyl, linear alkenyl, branched alkenyl or cycloalkenyl with 2 to 25 carbon atoms, whereby the alkenyl may contain from 1 to 10 non-conjugated double-bonds. Preferred are linear, branched or cyclic C₂₋₁₅-carbon-hydrogen moieties.

If R¹ and/or R³ is a linear, branched or "cyclic" alkenyl, the alkenyl preferably contains one non-conjugated double bond.

The term "non-conjugated double bond" means that the double bond is not in conjugation with the keto-group or the triple bond.

The term "linear C₁₋₄-alkyl" encompasses methyl, ethyl, n-propyl and n-butyl.

### Preferred starting material

### Preferred ketones

Preferred are ketones of the formula II, wherein R¹ is selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl, and/or wherein R² is methyl.

More preferred are ketones of the formula II, wherein R¹ is selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl, and wherein R² is methyl.

Preferably the ketone of the formula II is selected from the group consisting of methylethylketone (MEK; 2-butanone), isobutylmethylketone (IMK; 4-methylpentan-2-one), 6-methylhept-5-en-2-one (MH), (E,Z)-ethylheptenone ((E,Z)-EH; (E,Z)-3-methyloct-3-en-7-one), hexahydropseudoionone (HPI; 6,10-dimethylundecan-2-one), and C₁₈-ketone (C₁₈-K; 6,10,14-trimethylpentadecan-2-one).

### Preferred alkinols

Preferred are alkinols of the formula III, wherein R³ is selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl, and/or wherein R⁴ is methyl.

More preferred are ketones of the formula II, wherein R³ is selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl, and wherein R⁴ is methyl.

Preferably the alkinol of the formula III is selected from the group consisting of ethylbutinol (EBI; 3-hydroxy-3-methyl-1-pentine), 3,5-dimethylhexin-3-ol, dehydrolinalool (DLL; 3-hydroxy-3,7-dimethyloct-6-en-1-ine), (*E*,*Z*)-ethyldehydrolinalool (EDLL; (*E*,*Z*)-3-hydroxy-3,7-dimethyl-non-6-en-1-ine), C₁₅-acetylenalcohol (C₁₅-AA; 3-hydroxy-3,7,11-trimethyl-1-dodecine), and dehydroisophytol (DIP; 3-hydroxy-3,7,11,15-tetramethyl-1-hexadecine).

### Preferred products

Preferred are compounds of the formula I, wherein R² is methyl and/or wherein R⁴ is methyl, more preferably wherein both, R² and R⁴, are methyl.

Preferred are also compounds of the formula I, wherein R¹ and/or R³ are selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl, more preferably wherein both, R¹ and R³, are selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl.

Especially preferred are those compounds of the formula I, wherein R² and R⁴ are both methyl and wherein R¹ and R³ are selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl.

Preferred products are the following:
3,6-dihydroxy-3,6-dimethyl-4-octine,
4,7-dihydroxy-2,4,7,9-tetramethyl-5-decine,
6,9-dihydroxy-2,6,9,13-tetramethyl-2,12-tetradecadien-7-ine,
(*E*,*Z*)-7,10-dihydroxy-3,7,10,14-tetramethyl-3,13-hexadecadien-8-ine,
10,13-dihydroxy-2,6,10,13,17,21-hexamethyl-11-docosine, and 14,17-dihydroxy-2,6,10,14,17,21,25,29-octamethyl-15-triacontine.

Novel alkinediols of the formula I and, thus, also an object of the present invention are 4,7-dihydroxy-2,4,7,9-tetramethyl-5-decine, 10, 13-dihydroxy-2,6,10,13,17,21-hexamethyl-11-docosine and 14, 17-dihydroxy-2,6,10,14,17,21,25,29-octamethyl-15-triacontine.

### Preferred reaction conditions

Preferably the reaction is carried out at an absolute pressure of higher than 8 bar, preferably at an absolute pressure of 8 to 10 bar.

Preferably the reaction is carried out at a temperature of 10 to 60°C, preferably at a temperature of 21 to 40°C.

The process as claimed in any one of preceeding claims, characterized in that the molar ratio of the potassium hydroxide to the ketone of the formula II is less than 1 : 1, preferably between 1 : 10 and 1 : 1.

### Novel use

The present invention is also directed to the use of the alkinediols of the formula I with the preferences as given above as emulators, surfactants, dispersants, anti-foaming non-ionic agents, viscosity stabilizers, antistatic lubricants in the spinning of fibres, solvents, flavours, fragrances, wetting agents, sealing material or intermediate thereof.

The present invention is further illustrated by the following examples.

### Examples

The following abbreviations are used:
GC = gas chromatography

Before the reaction was carried out, the autoclave was tested under a pressure of 9 bar nitrogen (for 15 minutes). All equipment, which was in contact with ethine subsequently, was evacuated and filled with nitrogen (four cycles).

### Example 1: Preparation of 2,6,9,13-Tetramethyl-tetradeca-2,12-dien-7-yne-6,9-diol

In a 2-1 autoclave 369 g of ammonia were added to a mixture of 97 g of MH, 117 g of DLL and 92 g of aqueous KOH (47 weight-%) at 25°C. The reaction mixture was stirred with a velocity of 1200 rounds per minute. After a reaction time of 22 hours at 25°C the ammonia was evaporated, the residue acidified with aqueous sulphuric acid (8 weight-%), and washed with water of a temperature of 60°C under pH control (pH 7).

2,6,9,13-Tetramethyl-tetradeca-2,12-dien-7-yne-6,9-diol was isolated and purified from the crude mixture (250 g) by column chromatography on SiO₂ (2 kg). The product was eluted with a solvent mixture consisting of ethyl acetate and dichloromethane in a volume ratio of 9 : 1. During the chromatography the polarity of the eluent was changed to a solvent mixture containing ethyl acetate and dichloromethane in a volume ratio of 1 : 9 (after elution of remaining starting material). The resulting product fractions were collected and concentrated in vacuum (40°C, 600 to 10 mbar). The crude product was finally purified by crystallization from hexane (11) at 22°C (18.9 g), 4°C (13.27 g), -20°C (5.52 g). The fractions were combined, dried at 0.01 mbar for 24 hours at 20°C, and the purity was determined by GC (purity: 99.08 %). Yield 37.69 g.

Melting point: 56°C.
1H-NMR (CDCl₃) δ: 5.17 (t, 2H, =CH), 2.27 (m, 2H, =CH-CH₂-), 2.17 (m, 2H, =CH-CH₂-), 2.11 (s, 2H, OH), 1.68 (m, 16H, -CH₂-, =C-CH₃), 1.49 (s, 6H, CCH3).
13C-NMR (CDCl3) δ: 132.85 (=C-(CH₃)₂), 124.22 (=C-CH), 87.48 (C≡C), 68.74 (C-OH), 43.83 (C-CH₂-CH₂), 30.41 (C-CH₃), 26.13 (=C(CH₃)₂), 24.19 (=C-CH₂-CH₂), 18.14 (=C(CH₃)₂).

IR (Nujol) cm⁻¹: 3256 (broad), 2926 (broad), 2854, 2728,1676, 1454, 1376, 1233, 1121, 1085, 1020, 930, 893. Raman cm⁻¹: 2244.

MS (EI) m/z (%) = 245.1 (M - CH₃ and H₂O, 3), 227 (245 - H₂O, 4), 217.1 (M - H₂O and C₃H₇, 5), 69.1 (C₅H₉⁺, 71), 43.1 (C₂H₃O⁺, 100).

Microanalysis: calculated for C₁₈H₃₀O₂ (molecular weight 278.43): C 77.65, H 10.86; found: C 77.39, H 10.78.

By Karl-Fischer titration a content of 0.24 weight-% of water was found. In the product 0.03 weight-% of ammonia were detected by head-space GC.

### Example 2: Preparation of 2,6,10,13,17,21-Hexamethyl-docos-11-yne-10,13-diol

In a 2-1 autoclave 360 g of ammonia were added to a mixture of 77 g of HPI, 87 g of C₁₅-AA and 46 g of aqueous KOH (47 weight-%) at 25°C. The reaction mixture was stirred with a velocity of 1200 rounds per minute. After a reaction time of 22 hours at 25°C the ammonia was evaporated, the residue acidified with aqueous sulphuric acid (8 weight-%) and washed with water of a temperature of 60°C under pH control (pH 7).

2,6,10,13,17,21-Hexamethyl-docos-11-yne-10,13-diol was isolated and purified from the crude mixture (250 g) by column chromatography on SiO₂ (2 kg). The product was eluted with a solvent mixture consisting of ethyl acetate and dichloromethane in a volume ratio of 9:1. During the chromatography the polarity of the eluent was changed to a solvent mixture consisting of ethyl acetate and dichloromethane in a volume ratio of 1 : 9 (after elution of remaining starting materials). The resulting product fractions were collected and concentrated in vacuum (40°C, 600 to 10 mbar). This procedure was repeated twice to obtain pure material. The product was dried at 0.01 mbar for 24 h at 20°C and the purity was determined by GC (purity: 99.28%). Yield 22.35 g.

1H-NMR (CDC13): δ = 1.91 (s, 2H, OH), 1.47 (s, 6H, C-CH₃), 1.0 - 1.75 (m, 28H, CH and CH₂), 0.86 (d, 18H, CHCH₃).

13C-NMR (CDC13): δ = 87.46 (≡C), 68.62 (≡C-OH), 44.43, 39.78, 37.77, 37.51, 33.12, 30.33, 28.42, 25.23, 23.15, 23.04, 22.68, 20.00.

IR (thin film) cm⁻¹: 3373 (broad), 2953, 2869, 1463, 1367, 1251, 1150, 1112, 1065, 932, 896; Raman cm⁻¹: 2239.

MS (EI) m/z (%) = 405.3 (M - OH, 22), 389.3 (M - H₂O and CH₃, 7), 249.2 (M - C₁₁H₂₃ - H₂O, 100), 231.2 (249.2-H₂O, 28).

Microanalysis: calculated for C₂₈H₅₄O₂ (molecular weight 422.74): C 79.56, H 12.88; found: C 78.42, H 12.53.

By Karl-Fischer titration a content of 0.36 weight-% of water was found. In the product 0.01 weight-% of ammonia were detected by head-space GC.

### Example 3: Preparation of 2,6,10,14,17,21,25,29-Octamethyl-triacont-15-yne-14,17-diol

In a 2-1 autoclave 351 g of ammonia were added to a mixture of 96 g of C₁₈-K, 105 g of DIP and 43 g of aqueous KOH (47 weight-%) at 25°C. The reaction mixture was stirred with a velocity of 1200 rounds per minute. After 22 hours the ammonia was evaporated, the residue acidified with aqueous sulphuric acid (8 weight-%), and washed with water of a temperature of 60°C under pH control (pH 7).

2,6,10,14,17,21,25,29-Octamethyl-triacont-15-yne-14,17-diol was isolated and purified from the crude mixture (240 g) by column chromatography on SiO₂ (2 kg). The product was eluted with a solvent mixture consisting of ethyl acetate and dichlormethane in a volume ratio of 9 : 1. During the chromatography the polarity of the eluent was changed to a solvent mixture consisting of ethyl acetate and dichlormethane in a volume ratio of 1 : 9 (after elution of remaining starting materials). The resulting product fractions were collected and concentrated in vacuum (40°C, 600 to 10 mbar). This procedure was repeated twice to obtain pure material. The product was dried at 0.01 mbar for 24 hours at 20°C, and the purity was determined by GC (purity: 98.63%). Yield 91.37 g.

1H-NNM (CDCl₃): δ = 1.93 (s, 2H, OH), 1.47 (s, 6H, C-CH₃), 1.0 - 1.75 (m, 42H, CH₂ and CH), 0.83 - 0.89 (m, 24H, CH-CH₃).

13C-NMR (CDCl₃): δ = 87.46 (≡C), 68.61 (≡C-C-OH), 44.44, 39.81, 37.87, 37.74, 37.58, 37.48, 33.26, 33.14, 30.33, 28.41, 25.25, 24.93, 23.15, 23.06, 22.68, 20.18, 20.04.

IR (thin film) cm⁻¹: 3376 (broad), 2926, 2869, 1463, 1377, 1367, 1154, 1068, 931; Raman cm⁻¹: 2240.

MS (EI) m/z (%) = 529.5 (M - CH₃ and H₂O, 5), 319.3 (M - H₂O and C₁₆H₃₃, 100).

Microanalysis: calculated for C₃₈H₇₄O₂ (molecular weight 563.81): C 81.07, H 13.25; found: C 80.69, H 13.06.

By Karl-Fischer titration a content of 0.14 weight-% of water was found. In the product less than 0.01 weight-% of ammonia were detected by head-space GC.

### Example 4: Preparation of (E/Z)-3,7,10,14-Tetramethyl-hexadeca-3,13-dien-8-yne-7,10-diol

In a 2-1 autoclave 381 g of ammonia were added to a mixture of 83 g of EH, 99 g of EDLL and 71 g of aqueous KOH (47 weight-%) at 25°C. The reaction mixture was stirred with a velocity of 1200 rounds per minute. The reaction was stopped after 22 h by evaporating the ammonia, acidifying the residue with aqueous sulphuric acid (8 weight-%) and washing it with water of a temperature of 60°C under pH control (pH 7).

(E/Z)-3,7,10,14-Tetramethyl-hexadeca-3,13-dien-8-yne-7,10-diol was isolated and purified from the crude mixture (220 g) by column chromatography on SiO₂ (2 kg). The product was eluted with a solvent mixture consisting of ethyl acetate and dichloromethane in a volume ratio of 9 : 1. During the chromatography the polarity of the eluent was changed to a solvent mixture consisting of ethyl acetate and dichloromethane in a volume ration of 1 : 9 (after elution of the starting materials). The resulting product fractions were collected and concentrated in vacuum (40°C, 600 to 10 mbar). This procedure was repeated twice to obtain pure material. The product was dried at 0.01 mbar (24 h, 20°C), and the purity was determined by GC (purity: 97.93%). Yield 72.3 g.

1H-NMR (CDCl₃): δ = 5.15 (m, 2H, =CH), 2.0 - 2.4 (m, 10H, OH, CH₂-CH₃, and CH₂), 1.63 - 1.74 (m, 10H, =C-CH₃ and CH₂), 1.48 (s, 6H, C-CH₃), 0.98 (t, 6H, CH₂CH₃).

13C-NMR (CDCl₃): δ = 138.58 (CH₃-C=CH), 138.42 (CH₃-C=CH), 123.82 (=CH), 122.65 (=CH), 87.47 (≡C), 68.80 (C-OH), 68.74 (C-OH), 44.17 (C-CH₂), 43.86 (C-CH₂), 32.76 (CH₂-CH₃), 30.41 (CH₂-CH₃), 25.21 (CH₃COH), 24.04 (CH₃COH), 23.78 (=C-CH₂-CH₂), 23.31 (=C-CH₂-CH₂), 16.38 (=C-CH₃), 13.23 (CH₂-CH₃), 13.12 (CH₂-CH₃).

IR (film) cm⁻¹: 3377 (broad), 2966, 2933, 2875, 1667, 1457, 1373, 1263, 1242, 1166, 1124, 1092, 929, 848; Raman cm⁻¹: 1669, 2239.

MS (EI) m/z (%) = 273 (M - H₂O and CH₃, 2), 83.2 (C₆H₁₁⁺, 30), 55.1 (C₄H₇⁺, 100).

Microanalysis: calculated for C₂₀H₃₄O₂ (molecular weight 306.49): C 78.38, H 11.18; found: C 77.6, H 11.03.

By Karl-Fischer titration a content of 0.32 weight-% water was found. In the product less than 0.01 weight-% of ammonia was detected by head-space GC.

### Example 5: Preparation of 3,6-Dimethyl-oct-4-yne-3,6-diol

In a 2-1 autoclave 388 g of ammonia were added to a mixture of 77 g of MEK, 105 g of EBI and 127 g of aqueous KOH (47 weight-%) at 25°C. The reaction mixture was stirred with a velocity of 1200 rounds per minute. The reaction was stopped after 22 h by evaporating the ammonia, acidifying the residue with aqueous sulphuric acid (8 weight-%) and washing it with water of a temperature of 60°C under pH control (pH 7).

3,6-Dimethyl-oct-4-yne-3,6-diol was isolated and purified from the crude mixture (210 g) by column chromatography on SiO₂ (2 kg). The product was eluted with a solvent mixture consisting of ethyl acetate and dichloromethane in a volume ratio of 9 : 1. During the chromatography the polarity of the eluent was changed to a solvent mixture consisting of ethyl acetate and dichloromethane in a volume ration of 1 : 9 (after elution of the starting materials). The resulting product fractions were collected and concentrated in vacuum (40°C, 600 to 10 mbar). The crude product was finally purified by crystallization from hexane (11) at 22°C (43.36 g), 4°C (3.24 g), -20°C (3.31g). The fractions were combined, dried at 0.01 mbar for 24 hours at 20°C, and the purity was determined by GC (purity: 99.80 %). Yield 49.91 g.

Melting point: 52°C.

1H-NMR (CDCl₃): δ = 2.09 (s, 2H, OH), 1.68 (m, 4H, CH₂), 1.46 (s, 6H CCH₃), 1.03 (t, 6H, CH₂-CH₃).

13C-NMR (CDCl₃): δ = 87.27 (≡C), 69.05 (C-OH), 36.98 (CH₂CH₃), 29.78 (CCH₃), 9.47 (CH₂CH₃).

IR (Nujol) cm⁻¹ : 3245 (broad), 2926, 2853, 1464, 1376, 1334, 1272, 1162, 1129, 1033, 991, 915; Raman cm⁻¹: 2241.

MS (EI) m/z (%) = 141.1 (M - C₂H₅, 12), 137.1 (M - H₂O - CH₃, 6), 123.1 (M - H₂O -C₂H₅, 100), 43.2 (C₂H₃O⁺, 92).

Microanalysis: calculated for C₁₀H₁₈O₂ (molecular weight 170.25): C 70.55, H 10.66; found: C 70.03, H 10.43.

By Karl-Fischer titration a content of 0.30 weight-% of water was found. In the product 0.03 weight-% of ammonia was detected by head-space GC.

## Claims

1. A process for the manufacture of alkinediols of the formula I 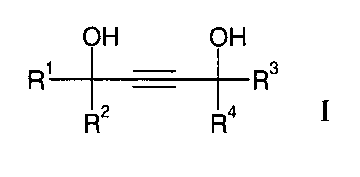 wherein ketones of the formula II 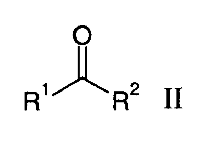 are reacted with alkinols of the formula III 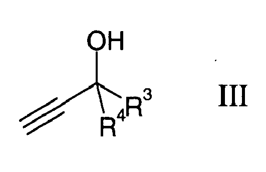 in liquid ammonia in the presence of potassium hydroxide, wherein R¹ and R³ are independently from each other a linear, branched or cyclic C₂₋₂₅-carbon-hydrogen moiety, optionally containing 1 to 10 non-conjugated double-bond(s), and R² and R⁴ are independently from each other linear C₁₋₄-alkyl.

2. The process as claimed in claim 1, **characterized in that** the reaction is carried out at an absolute pressure of higher than 8 bar, preferably at an absolute pressure of 8 to 10 bar.

3. The process as claimed in claim 1 and/or 2, **characterized in that** the reaction is carried out at a temperature of 10 to 60°C, preferably at a temperature of 21 to 40°C.

4. The process as claimed in any one of the preceding claims, **characterized in that** R² and R⁴ are both methyl.

5. The process as claimed in any one of the preceding claims, **characterized in that** R¹ and R³ are independently from each other selected from the group consisting of -CH₂-CHR⁵R⁶, wherein R⁵ is selected from the group consisting of hydrogen, methyl, -CH=C(CH₃)(CH₂)ₘCH₃ and -[CH₂-CH(CH₃)-(CH₂)₂-]ₙCH₂-CH(CH₃)₂ with m being 0 or 1 and n being 1, 2 or 3, and wherein R⁶ is hydrogen or methyl.

6. The process as claimed in any one of the preceding claims, **characterized in that** the molar ratio of the potassium hydroxide to the ketone of the formula II is less than 1 : 1, preferably between 1 : 10 and 1 : 1.

7. The process as claimed in any one of the preceding claims, **characterized in that** the ketone of the formula II is selected from the group consisting of methylethylketone, isobutylmethylketone, 6-methylhept-5-en-2-one, 3-methyloct-3-en-7-one, hexahydropseudoionone, and 6,10,14-trimethylpentadecan-2-one.

8. The process as claimed in any one of the preceding claims, **characterized in that** the alkinol of the formula III is selected from the group consisting of 3-hydroxy-3-methyl-1-pentine, 3,5-dimethylhexin-3-ol, dehydrolinalool, ethyldehydrolinalool, 3-hydroxy-3,7,11-trimethyl-1-dodecine, and dehydroisophytol.

9. Compound selected from the group consisting of 4,7-dihydroxy-2,4,7,9-tetramethyl-5-decine, 10, 13-dihydroxy-2,6,10,13,17,21-hexamethyl-11-docosine and 14, 17-dihydroxy-2,6,10,14,17,21,25,29-octamethyl-15-triacontine.

10. Use of the compounds of the formula I as defined in claim 1 as emulgator, surfactant, dispersant, anti-foaming non-ionic agent, viscosity stabilizer, antistatic lubricant in the spinning of fibres, solvent, flavour, fragrance, wetting agent, sealing material or intermediate thereof.
